# FASCICULE DE BREVET EUROPEEN

(11) **EP 1 712 207 B1**
(45) Date de publication et mention de la délivrance du brevet: **09.05.2012**
(21) Numéro de dépôt: 05008271.8
(22) Date de dépôt: 15.04.2005
(51) Int. Cl.: A61F 2/44

(54) **Prothèse de disque intervertébral**
Bandscheibenprothese
Intervertebral disc

(43) Date de publication de la demande: 18.10.2006
(73) Titulaire: Eden Spine Europe SA, 1207 Genève (CH)
(72) Inventeur: Lemaire, Jean-Philippe, 21910 Saulon la Chapelle (FR)
(74) Mandataire: Micheli & Cie SA

(56) Documents cités:
- WO-A-02/089701
- FR-A- 2 846 550
- US-A1- 2004 002 761

## Description

La présente invention concerne une prothèse destinée à remplacer le disque intervertébral.

Le disque intervertébral est un disque fibrocartilagineux qui assure la liaison entre deux vertèbres adjacentes de la colonne vertébrale. L'ensemble formé par le disque intervertébral et les deux vertèbres adjacentes correspondantes est appelé « segment vertébral ».

Les prothèses de disque intervertébral sont généralement constituées de deux plateaux dits supérieur et inférieur en regard l'un de l'autre et destinés à être fixés à deux vertèbres adjacentes respectives dites supérieure et inférieure, et d'un noyau interposé entre les deux plateaux. Les plateaux sont généralement métalliques et le noyau réalisé en métal ou en un polymère tel que le polyéthylène à haute densité. Deux types principaux de telles prothèses existent à ce jour :
- les prothèses dites non contraintes, dans lesquelles le noyau est libre par rapport aux deux plateaux et comporte des surfaces d'articulation, généralement convexes, coopérant avec des surfaces d'articulation, généralement concaves, des plateaux ;
- les prothèses dites contraintes, dans lesquelles le noyau est solidaire de l'un des deux plateaux et comporte une surface d'articulation, généralement convexe, coopérant à la manière d'une rotule avec une surface d'articulation, généralement concave, de l'autre plateau.

Dans le premier type de prothèse, le noyau a cinq degrés de liberté, trois en rotation et deux en translation, par rapport à un repère fixe. Lors d'un mouvement de flexion/extension (patient penché en avant/arrière respectivement) ou d'inclinaison latérale (patient penché sur le côté), le plateau supérieur s'incline par rapport au plateau inférieur en glissant sur le noyau en direction d'une extrémité de la prothèse. Ce mouvement d'inclinaison du plateau supérieur, qui peut être vu comme une combinaison d'une rotation autour d'un axe horizontal passant par le centre de gravité de l'ensemble formé par le plateau supérieur et la vertèbre supérieure et d'une translation vers l'extrémité précitée de la prothèse, entraîne une translation du noyau vers l'extrémité opposée de la prothèse, translation qui compense ainsi la translation du plateau supérieur vers la première extrémité. Lors d'un mouvement de torsion du patient, le plateau supérieur pivote par rapport au plateau inférieur dans le plan horizontal en s'articulant avec le noyau. Des moyens de couplage entre les plateaux et le noyau, tels que des plots centraux faisant saillie sur le noyau ou les plateaux et coopérant avec des cavités correspondantes formées dans les plateaux ou le noyau respectivement, sont dans certains cas prévus pour limiter l'amplitude des mouvements de la prothèse ou simplement éviter l'éjection du noyau. Des exemples de telles prothèses non contraintes sont décrits dans le brevet US 5 401 269 et les demandes de brevet US 200410002761, FR 2846550 et WO 021089701.

Dans le second type de prothèse, le noyau a trois degrés de liberté en rotation et aucun degré de liberté en translation. Un exemple de telles prothèses contraintes est décrit dans la demande de brevet FR 2 659 226.

Aucun de ces deux types de prothèses ne reproduit fidèlement la biomécanique naturelle du segment vertébral. Ces prothèses sont en effet fondées sur une conception linéaire de la biomécanique alors que la biomécanique naturelle du segment vertébral, plus précisément la fonction qui relie le déplacement du segment vertébral à la force appliquée, est non linéaire.

La présente invention vise à fournir une prothèse de disque intervertébral permettant de reproduire plus fidèlement la biomécanique naturelle du segment vertébral. A cette fin, il est proposé une prothèse de disque intervertébral telle que définie dans la revendication 1 annexée, des modes de réalisation particuliers étant définis dans les revendications dépendantes.

D'autres caractéristiques et avantages de la présente invention apparaîtront à la lecture de la description détaillée suivante faite en référence aux dessins annexés dans lesquels :
- la figure 1 est une vue en coupe sagittale d'une prothèse de disque intervertébral selon un premier mode de réalisation de l'invention, en position neutre ;
- les figures 2 et 3 sont des vues en perspective montrant respectivement un plateau supérieur et un plateau inférieur de la prothèse selon le premier mode de réalisation ;
- les figures 4a et 4b sont respectivement une vue en perspective de dessus et une vue arrière d'un noyau de la prothèse selon le premier mode de réalisation ;
- la figure 5a est une vue en coupe sagittale schématique de la prothèse selon le premier mode de réalisation en train d'effectuer une première phase d'un mouvement de flexion ;
- la figure 5b est une vue en coupe sagittale schématique de la prothèse selon le premier mode de réalisation dans une position correspondant à la fin de la première phase du mouvement de flexion ;
- les figures 6 et 7 sont des vues en coupe sagittale schématiques montrant la prothèse selon le premier mode de réalisation en train d'effectuer une seconde phase d'un mouvement de flexion, respectivement d'extension ;
- la figure 8 est une vue en perspective éclatée, partiellement coupée, d'une prothèse de disque intervertébral selon un second mode de réalisation de l'invention ;
- les figures 9 et 10 sont des vues en coupe sagittale et frontale, respectivement, montrant la prothèse selon le second mode de réalisation en position neutre ;
- les figures 11 et 12 sont des vues en coupe sagittale schématiques montrant la prothèse selon le second mode de réalisation en train d'effectuer une seconde phase d'un mouvement de flexion, respectivement d'extension ;
- les figures 13 et 14 sont des vues en coupe frontale schématiques montrant la prothèse selon le second mode de réalisation en train d'effectuer une seconde phase d'un mouvement d'inclinaison latérale vers la droite, respectivement vers la gauche ;
- la figure 15 est une vue en perspective éclatée d'une variante de la prothèse selon le second mode de réalisation ;
- la figure 16 est une vue en perspective de dessus d'un plateau inférieur faisant partie d'une autre variante de la prothèse selon le second mode de réalisation,
- les figures 17 et 18 sont des vues en perspective de dessus et de dessous respectivement, montrant une prothèse de disque intervertébral selon un troisième mode de réalisation de l'invention ; et
- la figure 19 montre la courbe du déplacement naturel d'une vertèbre en fonction de la force appliquée à celle-ci.

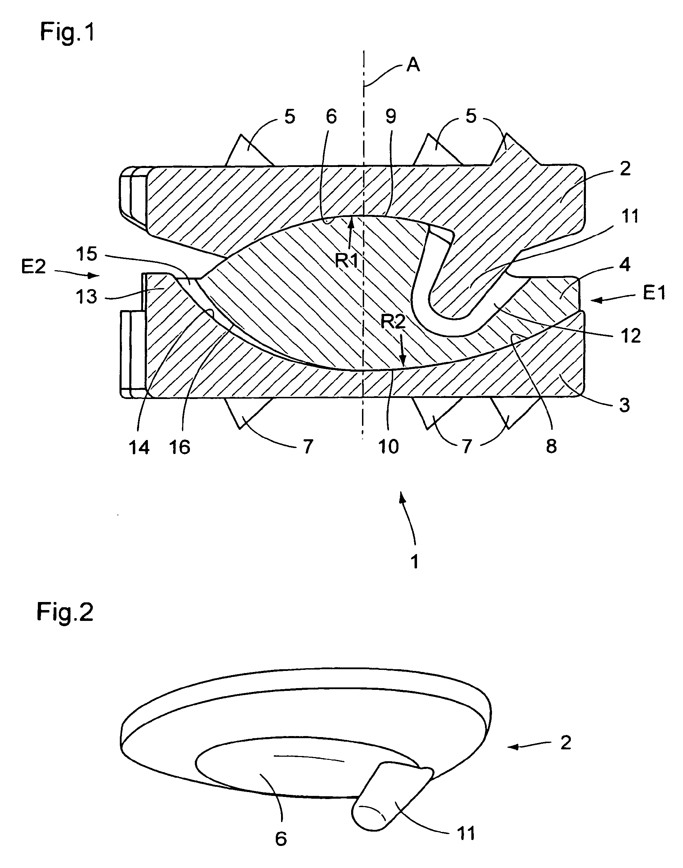

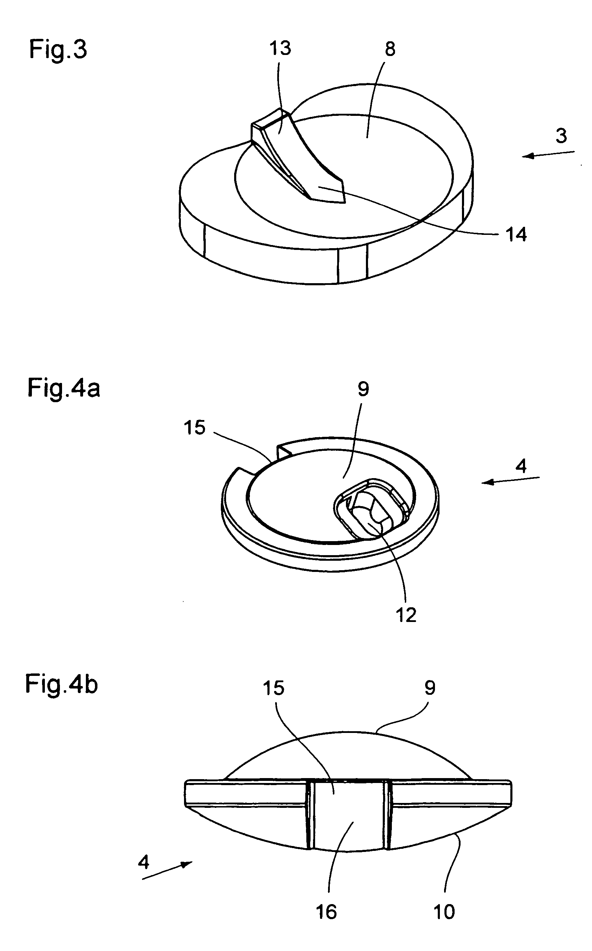

En référence aux figures 1 à 4, une prothèse de disque intervertébral 1 selon un premier mode de réalisation de l'invention comprend un plateau supérieur 2, un plateau inférieur 3 et un noyau 4. Le plateau supérieur 2 comporte sur sa face supérieure des pointes d'ancrage 5 pour sa fixation à une vertèbre dite supérieure et sur sa face inférieure une surface d'articulation concave 6, typiquement en forme de portion sphérique. Le plateau inférieur 3 comporte sur sa face inférieure des pointes d'ancrage 7 pour sa fixation à une vertèbre dite inférieure, adjacente à la vertèbre supérieure, et sur sa face supérieure une surface d'articulation concave 8, typiquement en forme de portion sphérique. Le noyau 4 est interposé entre les plateaux 2, 3 et comporte, respectivement sur ses faces supérieure et inférieure, deux surfaces d'articulation convexes opposées 9, 10 coopérant avec les surfaces d'articulation concaves 6, 8 des plateaux 2, 3. Chacune des deux surfaces d'articulation 9, 10 du noyau 4 est congruente avec la surface d'articulation correspondante 6, 8 du plateau 2, 3. De préférence, pour une raison qui sera indiquée plus loin, le rayon de courbure R1 des surfaces d'articulation 6, 9 est inférieur au rayon de courbure R2 des surfaces d'articulation 8, 10. Les plateaux 2, 3 sont par exemple en un métal du type alliage Co-Cr-Mb ou titane, et le noyau 4 en métal ou en un polymère du type polyéthylène à haute densité.

Le noyau 4 est couplé aux plateaux 2, 3 respectivement par des premiers et seconds moyens de couplage. Les premiers moyens de couplage comprennent une quille 11 faisant saillie sur une partie périphérique antérieure de la face inférieure du plateau supérieur 2 et une cavité 12 formée dans le noyau 4 et débouchant sur la surface d'articulation 9 de ce dernier, la cavité 12 recevant la quille 11. Par « partie périphérique » on entend une partie non traversée par l'axe A de la prothèse. La quille 11 est par ailleurs située dans le plan sagittal passant par l'axe A, et est inclinée par rapport au plateau supérieur 2 et dirigée vers ledit axe A. La quille 11 peut être formée d'un seul tenant avec le plateau supérieur 2 ou fixée à celui-ci par exemple par soudage. La cavité 12 a sensiblement la même forme, par exemple tronconique, que la quille 11, mais sa section est plus grande que celle de la quille 11 de façon à autoriser un débattement de la quille 11 et donc du plateau supérieur 2 par rapport au noyau 4 dans les plans sagittal, frontal et coronal. Les seconds moyens de couplage comprennent un rail 13 formé d'un seul tenant avec le plateau inférieur 3 ou fixé à celui-ci par exemple par soudage et s'étendant dans une direction sagittale sur la surface d'articulation 8 dudit plateau inférieur 3. Ce rail 13, situé dans la partie postérieure de la prothèse, définit une surface d'appui pentue 14 reliant le sommet du rail 13, au niveau du bord postérieur du plateau inférieur 3, à la partie centrale de la surface d'articulation 8 du plateau 3. Ce rail 13 coopère avec une rainure 15 de forme correspondante, comprenant elle aussi une surface d'appui pentue 16, formée dans la surface d'articulation inférieure 10 du noyau 4. La rainure 15 a une section plus large que celle du rail 13, de façon à autoriser un débattement en rotation axiale, c'est-à-dire autour de l'axe A, du noyau 4 par rapport au plateau inférieur 3 (torsion) ainsi qu'en inclinaison latérale.

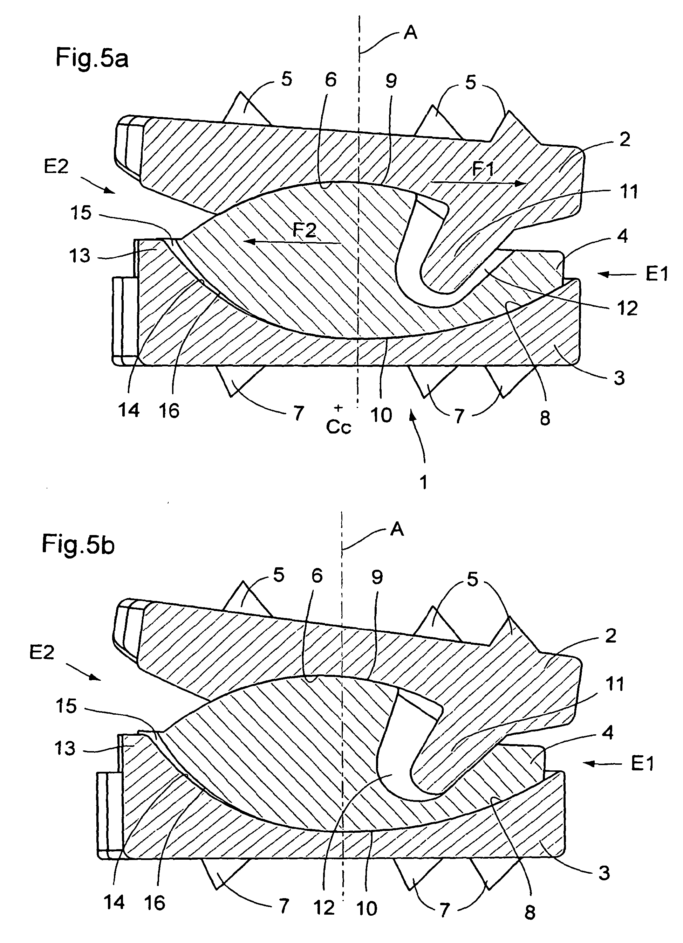

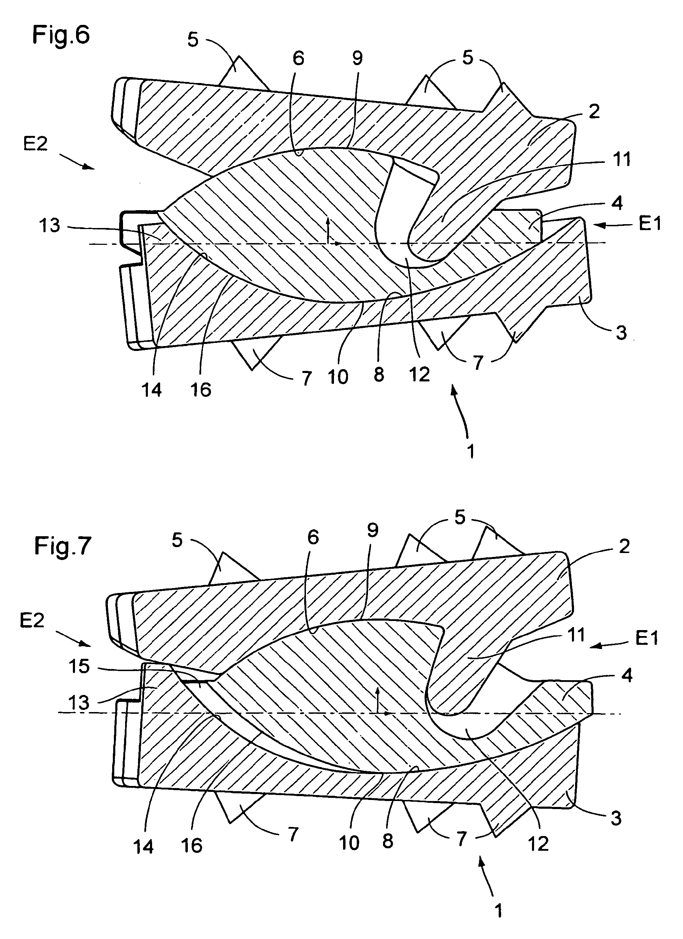

Conformément à l'invention, les mouvements d'inclinaison du plateau supérieur 2 de la prothèse par rapport au plateau inférieur 3 dans le plan sagittal (flexion / extension) ou frontal (inclinaison latérale) se décomposent en deux phases :
- une première phase au cours de laquelle le plateau supérieur 2 glisse sur le noyau 4, la quille 11 se déplaçant librement dans la cavité 12 ; et
- une seconde phase, qui débute juste après que la quille 11 est entrée en contact avec la paroi interne de la cavité 12 (cf. figure 5b), au cours de laquelle le plateau supérieur 2 entraîne avec lui le noyau 4 par l'intermédiaire de la quille 11 (cf. figure 6 : prothèse en flexion ; figure 7 : prothèse en extension).
Lors de la première phase, le plateau supérieur 2, avec la vertèbre dite supérieure auquel il est fixé, effectue un mouvement de rotation autour du centre de courbure Cc des surfaces d'articulation 6, 9. Ce mouvement de rotation tend à déplacer le plateau supérieur 2 vers une extrémité E1 de la prothèse, comme montré par la flèche F1 sur la figure 5a. Toutefois, comme le noyau 4 est libre par rapport aux plateaux 2, 3, le plateau supérieur 2, en glissant sur le noyau 4 vers l'extrémité E1, chasse le noyau 4 vers l'extrémité opposée E2 de la prothèse, comme montré par la flèche F2, ce qui déplace le centre de rotation Cc du plateau supérieur 2 vers l'extrémité E2 et compense le déplacement précité du plateau supérieur 2 vers l'extrémité E1. Ce mouvement de la prothèse pendant cette première phase est identique à celui d'une prothèse non contrainte. Il peut être vu comme une combinaison d'une rotation de l'ensemble plateau supérieur 2 - vertèbre supérieure autour d'un axe horizontal passant par son centre de gravité et d'une translation de cet ensemble, cette combinaison de mouvements imitant le mouvement combiné naturel rotation-translation de la vertèbre supérieure. Lors de la seconde phase, le plateau supérieur 2 et le noyau 4 forment un ensemble solidaire qui s'articule à la manière d'une rotule avec le plateau inférieur 3, c'est-à-dire tourne autour du centre de courbure des surfaces d'articulation 8, 10 (figures 6,7).

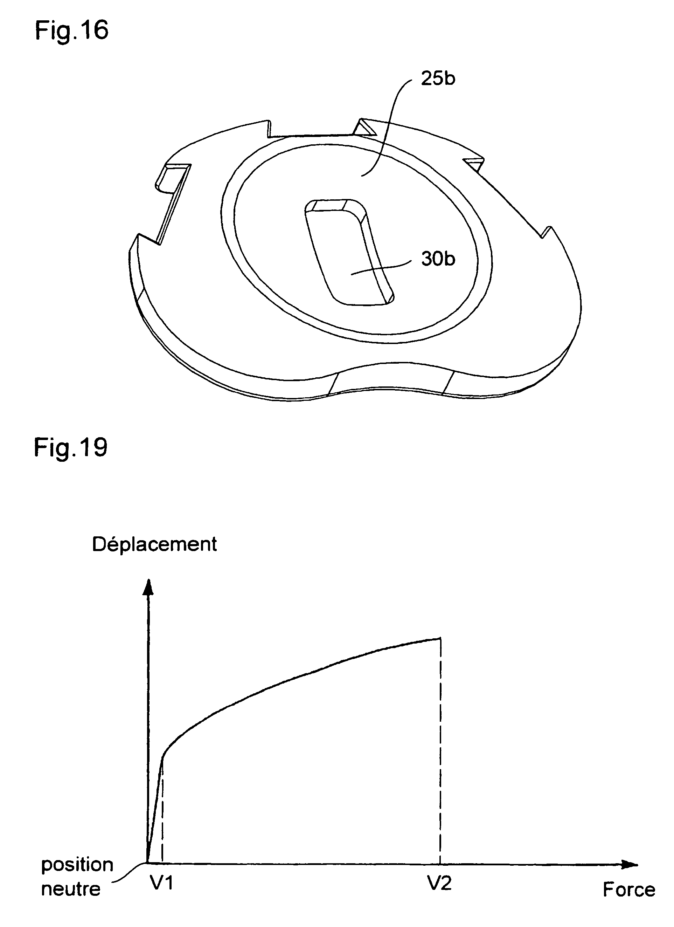

Lors de la première phase, une faible force appliquée à l'ensemble plateau supérieur 2 - vertèbre supérieure suffit à entraîner un déplacement d'inclinaison important de cet ensemble. Dans la seconde phase, un supplément de force bien plus élevé doit être appliqué pour obtenir la même amplitude de déplacement que dans la première phase, du fait que le plateau supérieur 2 entraîne le noyau 4 et doit donc exercer un effort plus important. Ceci est conforme à la biomécanique naturelle du segment vertébral. En effet, comme représenté à la figure 19, la courbe du déplacement d'inclinaison physiologique d'une vertèbre en fonction de la force appliquée à cette dernière présente une première partie linéaire correspondant à des forces allant de 0 à une valeur V1 et présentant une pente élevée, et une seconde partie non linéaire correspondant à des forces allant de la valeur V1 à la force physiologique maximale V2 et dont la pente est bien plus faible que celle de la partie linéaire et décroissante. Dans la présente invention, la première phase du mouvement d'inclinaison du plateau supérieur 2 par rapport au plateau inférieur 3 correspond sensiblement à la partie linéaire de la courbe précitée tandis que la seconde phase de ce mouvement correspond sensiblement à la partie non linéaire de cette courbe. Les valeurs V1 et V2 varient en fonction du patient. Les étendues angulaires respectives des première et seconde phases du mouvement de la prothèse doivent donc être adaptées au patient, en jouant sur les sections respectives de la quille 11 et de la cavité 12. Dans des exemples typiques, mais non limitatifs, de réalisation de la présente invention, la première phase correspond à une étendue angulaire d'inclinaison du plateau supérieur 2 par rapport au plateau inférieur 3, depuis sa position neutre montrée à la figure 1, d'environ 4 à 8°, de préférence d'environ 5 à 7°, et la seconde phase à une étendue angulaire d'inclinaison du plateau supérieur 2 par rapport au plateau inférieur 3 d'environ 3 à 7°, de préférence d'environ 4 à 6°. La première phase peut typiquement représenter environ 40 à 60% de l'angle maximum d'inclinaison du plateau supérieur 2 par rapport au plateau inférieur 3, et la seconde phase les quelque 60 à 40% restants.

Un autre avantage de ce mouvement en deux phases du plateau supérieur 2 par rapport au plateau inférieur 3 est qu'il permet de contrôler le déplacement du noyau 4, et ainsi limiter les contraintes, en particulier les hyper-pressions surfaciques ou ponctuelles et les hypo-pressions, pouvant s'exercer sur le système articulaire postérieur, du fait que ce noyau 4 est rendu solidaire du plateau supérieur 2 lors de la seconde phase.

On notera de plus que les moyens de couplage 11, 12, 13, 15 de la prothèse selon l'invention n'autorisent les mouvements de torsion (rotation axiale du plateau supérieur 2 par rapport au plateau inférieur 3) que dans des limites déterminées. Ceci contribue également à protéger le système articulaire postérieur.

Outre leur fonction de limitation de la rotation axiale du noyau 4 par rapport au plateau inférieur 3, le rail 13 et la rainure correspondante 15 coopèrent lors des mouvements de flexion pour soulever légèrement le centre de gravité du noyau 4, à l'instar du mouvement physiologique, par un glissement de la surface d'appui pentue 16 de la rainure 15 sur la surface d'appui pentue 14 du rail 13 (cf. figure 6).

Par ailleurs, du fait que les surfaces d'articulation congruentes 6, 9 ont un rayon de courbure R1 inférieur au rayon de courbure R2 des surfaces d'articulation congruentes 8, 10, la composante de rotation des mouvements d'inclinaison du plateau supérieur 2 est privilégiée par rapport à la composante de translation.

La prothèse selon la présente invention présente en outre l'avantage de limiter les risques d'éjection du noyau 4, grâce notamment à la coopération entre la quille 11 et la cavité 12.

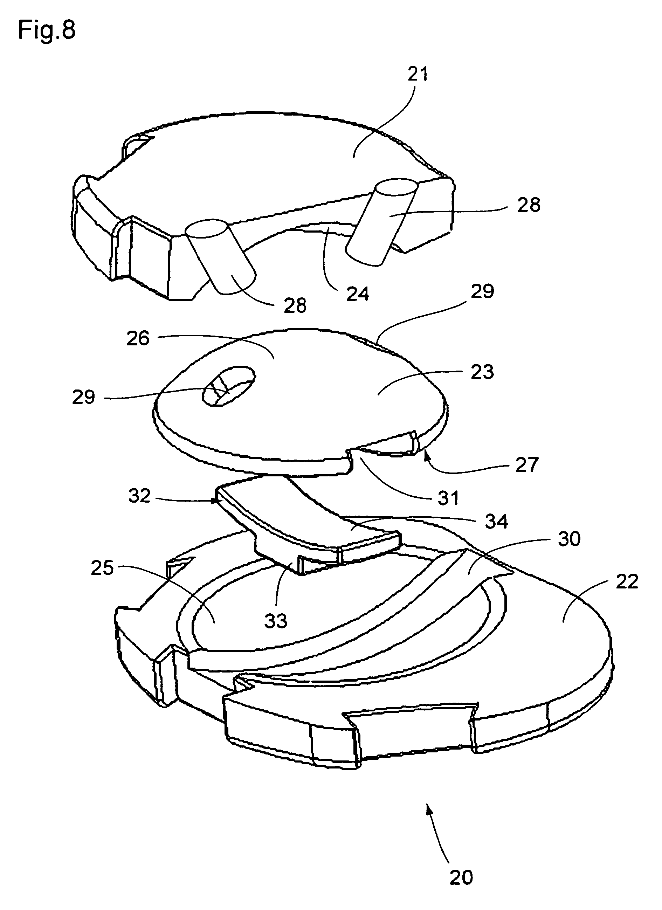

En référence aux figures 8 à 10, une prothèse de disque intervertébral 20 selon un second mode de réalisation de l'invention comprend des plateaux supérieur et inférieur 21, 22 destinés à être fixés à deux vertèbres adjacentes, et un noyau 23 interposé entre les plateaux 21, 22. Comme dans le premier mode de réalisation, les plateaux supérieur et inférieur 21, 22 comportent chacun une surface d'articulation concave 24, 25 coopérant avec une surface d'articulation convexe correspondante 26, 27 du noyau 23. Le plateau supérieur 21 et le noyau 23 sont couplés par des premiers moyens de couplage comprenant deux tétons inclinés 28 faisant saillie sur une partie périphérique de la surface d'articulation 24 du plateau supérieur 21 et deux cavités inclinées 29 formées dans la surface d'articulation 26 du noyau 23 et recevant les tétons 28. Les tétons 28 sont situés dans le plan sagittal passant par l'axe de la prothèse, dirigés vers ledit axe et disposés de part et d'autre de cet axe symétriquement l'un de l'autre. Les tétons 28 sont fixés dans le plateau supérieur 21 par exemple par soudage. Les cavités correspondantes 29 ont une plus grande section que celle des tétons 28 afin d'autoriser un débattement du plateau supérieur 21 par rapport au noyau 23 dans les plans sagittal, frontal et coronal.

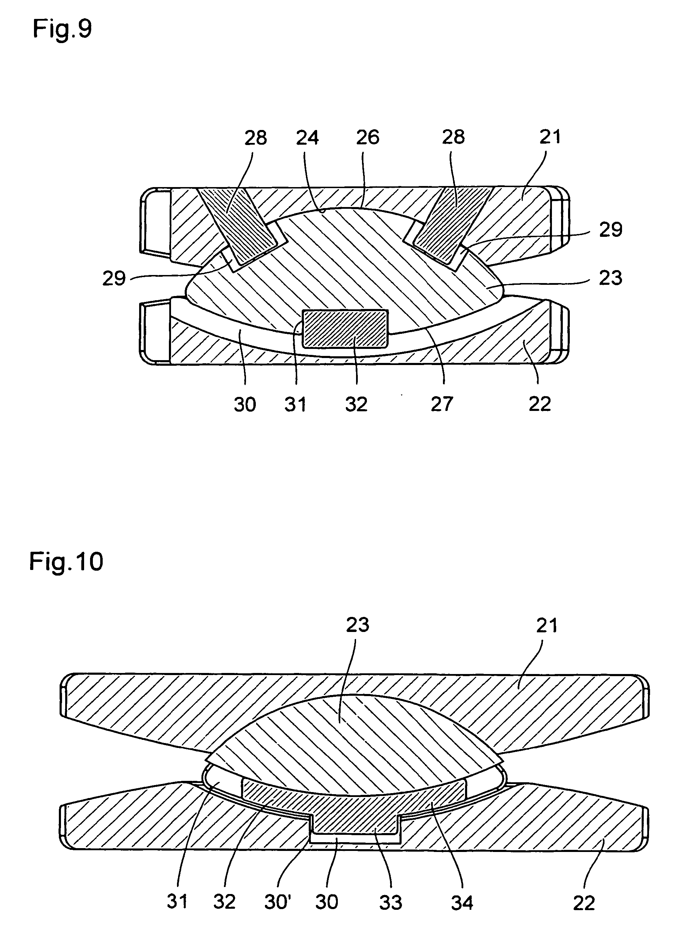

Le noyau 23 et le plateau inférieur 22 sont couplés par des seconds moyens de couplage comprenant une première rainure à section rectangulaire 30 formée dans la surface d'articulation 25 du plateau inférieur 22 et s'étendant dans une direction sagittale, une seconde rainure à section rectangulaire 31 formée dans la surface d'articulation 27 du noyau 23 et s'étendant dans une direction frontale, et une pièce intermédiaire mobile, de guidage, en forme de T 32 coopérant avec les deux rainures 30, 31. Plus précisément, la branche verticale 33 de la pièce de guidage 32, plus courte que la branche horizontale 34, est guidée dans la première rainure 30 tandis que la branche horizontale 34 est guidée dans la seconde rainure 31. La première rainure 30 est plus large que la branche verticale 33 de la pièce de guidage 32, de façon à définir un jeu 30' (visible uniquement sur la figure 10) autorisant un débattement en rotation axiale entre le noyau 23 et le plateau inférieur 22 (torsion).

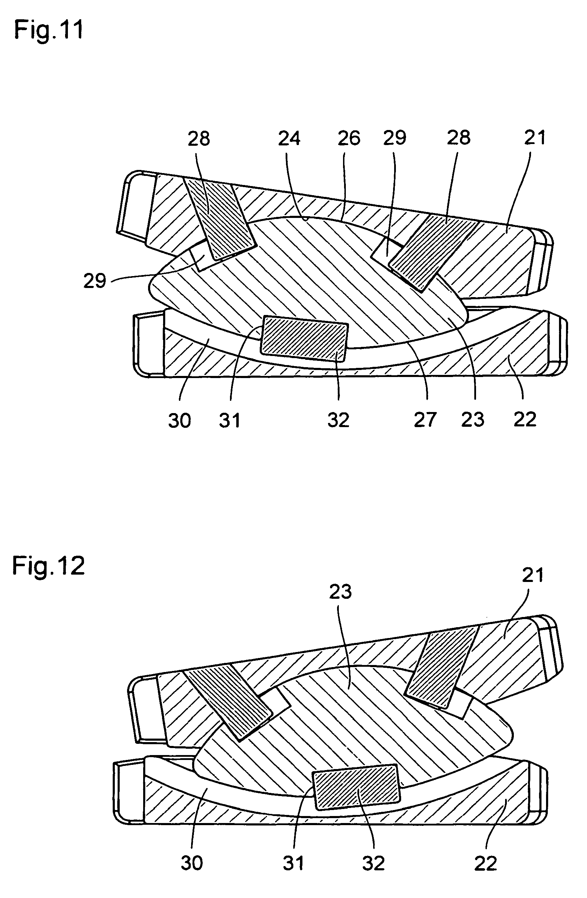

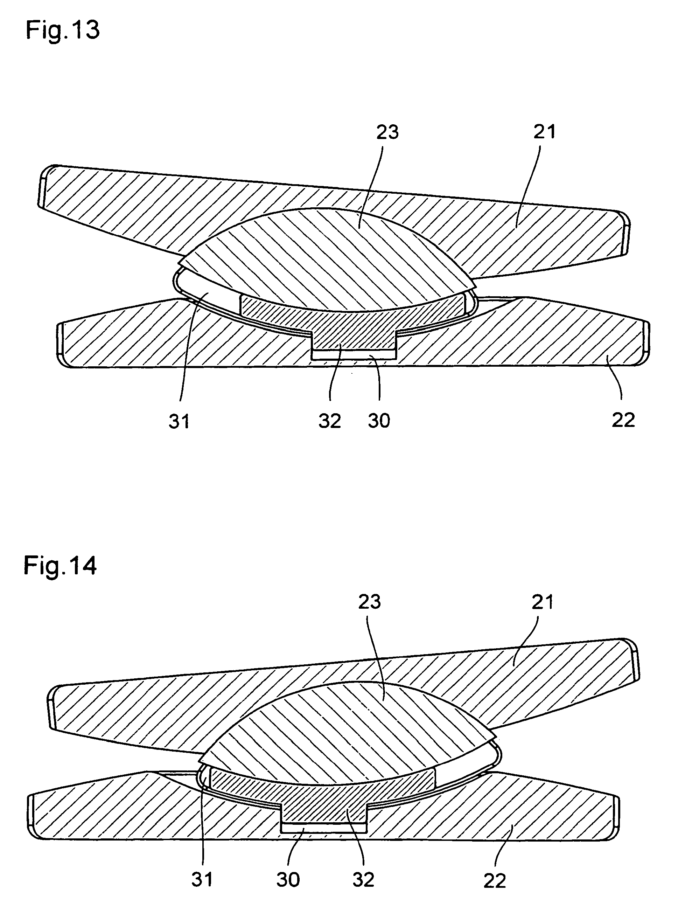

Les mouvements de flexion, extension et inclinaison latérale de la prothèse 20 sont similaires à ceux de la prothèse 1, c'est-à-dire qu'ils s'effectuent en deux phases, à savoir une première phase où le mouvement entre le plateau supérieur 21 et le noyau 23 est non contraint, les tétons 28 se déplaçant librement dans les cavités 29, et une seconde phase où les tétons 28, en butée contre la paroi interne de leurs cavités respectives 29, poussent le noyau 23 le rendant ainsi solidaire du plateau supérieur 21 (cf. figure 11 : flexion ; figure 12 : extension ; figure 13 : inclinaison latérale côté droit ; figure 14 : inclinaison latérale côté gauche). Lors des mouvements de flexion/extension, la pièce de guidage 32, guidée par la rainure 30, se déplace avec le noyau 23 (figures 11, 12). Lors des mouvements d'inclinaison latérale, la pièce de guidage 32 reste fixe par rapport au plateau inférieur 22 et guide le déplacement du noyau 23 par sa coopération avec la rainure 31 (figures 13, 14). Les mouvements de torsion de la prothèse 20 (rotation axiale du plateau supérieur 21 par rapport au plateau inférieur 22) sont limités par les moyens de couplage 28-32.

La prothèse 20 selon ce second mode de réalisation présente l'avantage, par rapport à la prothèse 1 selon le premier mode de réalisation, de mieux contrôler les mouvements de la prothèse, et donc de réduire les contraintes exercées sur le système articulaire postérieur, en limitant les mouvements couplés (combinaisons de mouvements de flexion/extension, d'inclinaison latérale et de rotation axiale), ceci grâce à la pièce de guidage 32 et aux rainures associées 30, 31 qui guident les mouvements de la prothèse.

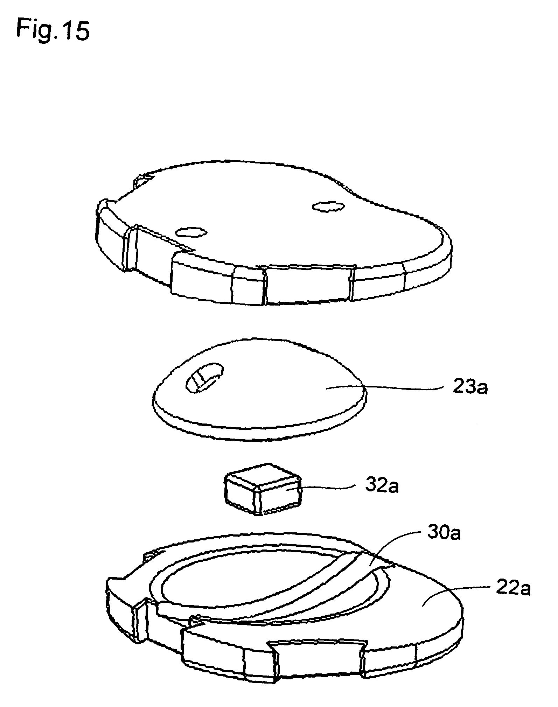

Dans une variante de réalisation de ce second mode de réalisation, montrée à la figure 15, la pièce de guidage 32a a une forme de parallélépipède rectangle dont la partie inférieure est reçue dans une rainure 30a du plateau inférieur 22a orientée sagittalement et la partie supérieure est reçue dans une rainure (non visible) du noyau 23a orientée frontalement.

La figure 16 montre une variante du plateau inférieur 22 de la prothèse selon le second mode de réalisation, dans laquelle la rainure sagittale 30b est fermée à ses deux extrémités et est tout entière à l'intérieur du contour de la surface d'articulation 25b pour limiter les déplacements de la pièce de guidage 32 ou 32a dans la direction sagittale.

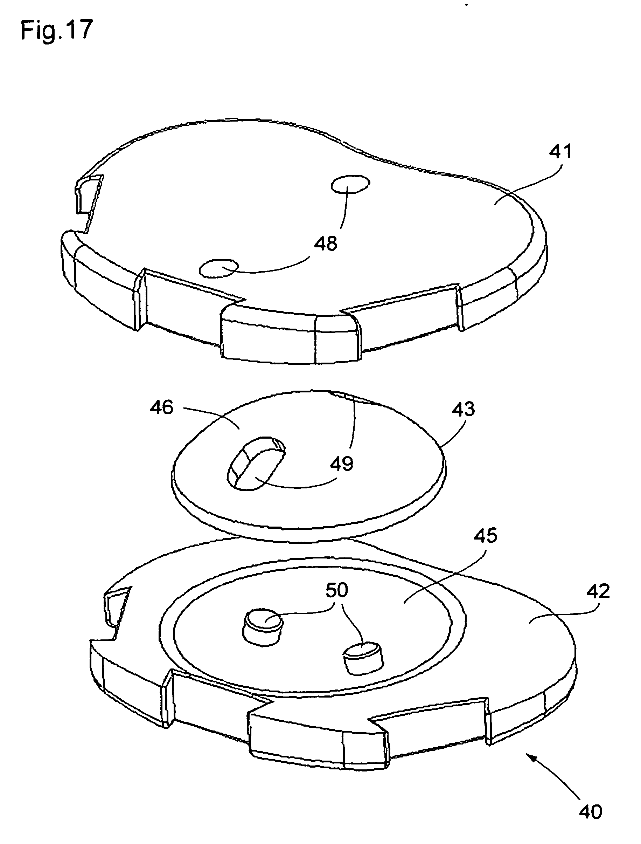

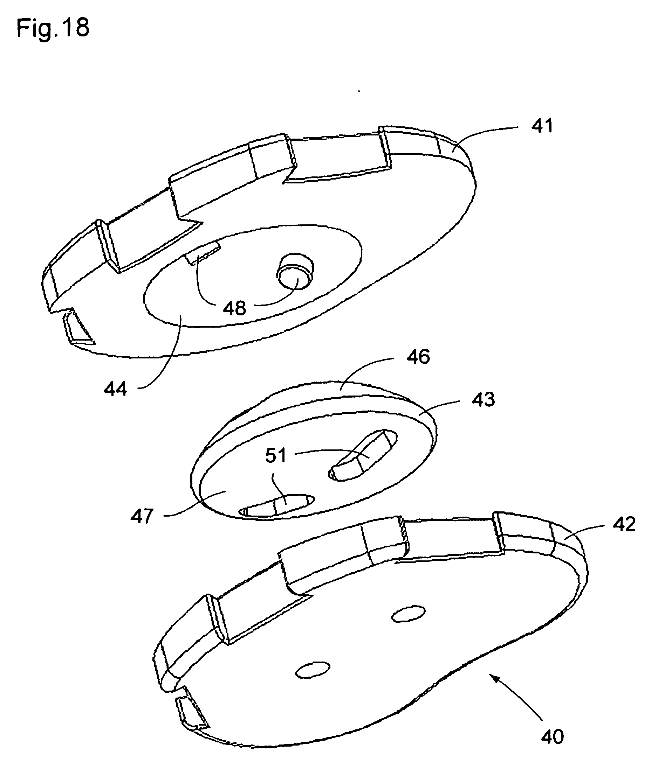

Les figures 17 et 18 montrent une prothèse de disque intervertébral 40 selon un troisième mode de réalisation de la présente invention. La prothèse 40 comprend un plateau supérieur 41, un plateau inférieur 42 et un noyau 43. Les plateaux 41, 42 comportent des surfaces d'articulation concaves respectives 44, 45 coopérant avec des surfaces d'articulation convexes respectives 46, 47 du noyau 43. Le plateau supérieur 41 et le noyau 43 sont couplés par des moyens de couplage comprenant, comme dans le second mode de réalisation, des tétons inclinés 48 faisant saillie sur une partie périphérique de la surface d'articulation 44 du plateau supérieur 41 et des cavités inclinées correspondantes 49 formées dans la surface d'articulation 46 du noyau 43. Les tétons 48 sont situés dans le plan sagittal passant par l'axe de la prothèse, dirigés vers ledit axe et symétriques l'un de l'autre par rapport audit axe. Les cavités correspondantes 49 ont ici une forme oblongue orientée dans une direction sagittale.

La prothèse 40 comporte en outre des moyens de couplage du noyau 43 et du plateau inférieur 42, constitués par des tétons inclinés 50 faisant saillie sur une partie périphérique de la surface d'articulation 45 du plateau inférieur 42 et des cavités inclinées correspondantes 51 formées dans la surface d'articulation 47 du noyau 43. Les tétons 50 sont situés dans le plan frontal passant par l'axe de la prothèse, dirigés vers ledit axe et symétriques l'un de l'autre par rapport audit axe. Les cavités correspondantes 49 ont une forme oblongue orientée dans une direction frontale.

Les jeux respectifs entre les tétons 48 et les cavités 49 et entre les tétons 50 et les cavités 51 sont suffisamment grands pour permettre des mouvements de flexion/extension ou d'inclinaison latérale du plateau supérieur 41 par rapport au plateau inférieur 42 en deux phases, à savoir une première phase pendant laquelle les tétons 48 se déplacent librement dans les cavités 49 et une seconde phase pendant laquelle le plateau supérieur 41 entraîne le noyau 43 par l'intermédiaire des tétons 48. Pendant ces mouvements d'inclinaison du plateau supérieur 41, les moyens de couplage 50, 51 guident les mouvement du noyau 43. Ces moyens de couplage 50, 51, avec les moyens 48, 49, limitent en outre la rotation axiale du plateau supérieur 41 par rapport au plateau inférieur 42.

La présente invention a été décrite ci-dessus à titre d'exemple uniquement. Il apparaîtra clairement à l'homme du métier que des modifications peuvent être faites sans sortir du cadre de l'invention. Par exemple, les parties saillantes 11, 28, 48 pourraient être prévues sur le noyau 4, 23, 43 plutôt que sur le plateau supérieur 2, 21, 41 et les cavités correspondantes 12, 29, 49 formées dans le plateau supérieur 2, 21, 41 plutôt que dans le noyau 4, 23, 43. De façon comparable, le rail 13 ou les tétons 50 pourraient être prévus sur le noyau 4, 43 plutôt que sur le plateau inférieur 3, 42 et la rainure correspondante 15 ou les cavités correspondantes 51 formées dans le plateau inférieur 3, 42 plutôt que dans le noyau 4, 43. Une autre modification de la prothèse selon l'invention pourrait consister à inverser les plateaux supérieur et inférieur. Dans ce cas, les premiers moyens de couplage 11-12, 28-29, 48-49 seraient associés à la surface inférieure du noyau, et les seconds moyens de couplage 13, 15, 30-32, 50-51 à la surface supérieure du noyau. Le rayon de courbure de la surface d'articulation supérieure du noyau et de la surface d'articulation correspondante du plateau supérieur resterait toutefois inférieur au rayon de courbure de la surface d'articulation inférieure du noyau et de la surface d'articulation correspondante du plateau inférieur.

## Revendications

1. Prothèse de disque intervertébral comprenant :
- des première et seconde pièces (2, 3) destinées à être fixées respectivement à deux vertèbres adjacentes,
- une pièce intermédiaire (4) interposée entre les première et seconde pièces (2, 3) et comprenant des surfaces d'articulation (9, 10) coopérant avec des surfaces d'articulation respectives (6, 8) des première et seconde pièces (2, 3), et
- des moyens (11, 12) de couplage de la première pièce (2) et de la pièce intermédiaire (4),
**caractérisée en ce que** les moyens de couplage (11, 12) sont agencés pour laisser la pièce intermédiaire (4) libre par rapport à la première pièce (2) lors d'une première phase d'un mouvement d'inclinaison de la première pièce (2) par rapport à la seconde pièce (3), de sorte que la pièce intermédiaire (4) se déplace vers une extrémité (E2) de la prothèse opposée à une extrémité (E1) vers laquelle la première pièce (2) se déplace en s'inclinant par rapport à la seconde pièce (3), et pour entraîner la pièce intermédiaire (4) avec la première pièce (2) lors d'une seconde phase du mouvement d'inclinaison.

2. Prothèse de disque intervertébral selon la revendication 1, **caractérisée en ce que** lesdits moyens de couplage comprennent au moins une partie saillante (11) prévue sur l'une de la première pièce (2) et de la pièce intermédiaire (4) et au moins une cavité (12) formée dans l'autre de la première pièce (2) et de la pièce intermédiaire (4), cette cavité (12) recevant la partie saillante (11) et ayant une plus grande section que celle de la partie saillante (11).

3. Prothèse de disque intervertébral selon la revendication 2, **caractérisée en ce que** lesdits moyens de couplage comprennent une unique partie saillante (11) prévue sur une partie périphérique de la première pièce (2) ou de la pièce intermédiaire (4) et dans un plan sagittal.

4. Prothèse de disque intervertébral selon la revendication 2, **caractérisée en ce que** lesdits moyens de couplage comprennent des première et seconde parties saillantes (28 ; 48) prévues sur la première pièce (21 ; 41) ou la pièce intermédiaire (23 ; 43) et situées dans un plan sagittal, de part et d'autre de l'axe de la prothèse, et des première et seconde cavités correspondantes (29 ; 49) formées dans la pièce intermédiaire (23 ; 43) ou la première pièce (21 ; 41) respectivement.

5. Prothèse de disque intervertébral selon l'une quelconque des revendications 1 à 4, **caractérisée en ce qu'**elle comprend en outre des moyens (13, 15) de couplage de la pièce intermédiaire (4) et de la seconde pièce (3) limitant les mouvements relatifs en rotation axiale desdites pièce intermédiaire (4) et seconde pièce (3).

6. Prothèse de disque intervertébral selon la revendication 5, **caractérisée en ce que** lesdits moyens de couplage de la pièce intermédiaire (4) et de la seconde pièce (3) comprennent un rail (13) s'étendant dans une direction sagittale sur l'une de la seconde pièce (3) et la pièce intermédiaire (4) et une rainure (15) formée dans l'autre de la seconde pièce (3) et la pièce intermédiaire (4) et coopérant avec ce rail (13).

7. Prothèse de disque intervertébral selon la revendication 6, **caractérisée en ce que** le rail (13) et la rainure (15) définissent des surfaces d'appui respectives pentues (14, 16) qui coopèrent lors de mouvements de flexion de la prothèse pour soulever légèrement le centre de gravité de la pièce intermédiaire (4).

8. Prothèse de disque intervertébral selon la revendication 5, **caractérisée en ce que** lesdits moyens de couplage de la pièce intermédiaire (23) et de la seconde pièce (22) comprennent une première rainure (30 ; 30a ; 30b) formée dans la seconde pièce (22) et orientée dans une direction sagittale, une seconde rainure (31) formée dans la pièce intermédiaire (23) et orientée dans une direction frontale, et une pièce de guidage mobile (32 ; 32a) coopérant avec les première et seconde rainures (30 ; 30a ; 30b, 31).

9. Prothèse de disque intervertébral selon la revendication 8, **caractérisée en ce qu'**un jeu (30') existe entre la pièce de guidage (32) et l'une au moins des première et seconde rainures (30, 31) permettant un débattement en rotation axiale de la pièce intermédiaire (23) par rapport à la seconde pièce (22).

10. Prothèse de disque intervertébral selon la revendication 5, **caractérisée en ce que** lesdits moyens de couplage de la pièce intermédiaire (43) et de la seconde pièce (42) comprennent des premier et second tétons (50) prévus sur la seconde pièce (42) ou la pièce intermédiaire (43) et situés dans un plan frontal, de part et d'autre de l'axe de la prothèse, et des troisième et quatrième cavités (51) formées dans la pièce intermédiaire (43) ou la seconde pièce (42), respectivement, et recevant les tétons (50).

11. Prothèse de disque intervertébral selon l'une quelconque des revendications 1 à 10, **caractérisée en ce que** la première phase du mouvement d'inclinaison représente environ 40 à 60% de l'angle maximum d'inclinaison de la première pièce (2) par rapport à la seconde pièce (3), et la seconde phase représente les quelque 60 à 40% restants.

12. Prothèse de disque intervertébral selon l'une quelconque des revendications 1 à 11, **caractérisée en ce que** la première phase du mouvement d'inclinaison correspond à une étendue angulaire d'inclinaison de la première pièce (2) par rapport à la seconde pièce (3) d'environ 4 à 8° et la seconde phase du mouvement d'inclinaison correspond à une étendue angulaire d'inclinaison de la première pièce (2) par rapport à la seconde pièce (3) d'environ 3 à 7°.

13. Prothèse de disque intervertébral selon l'une quelconque des revendications 1 à 11, **caractérisée en ce que** la première phase du mouvement d'inclinaison correspond à une étendue angulaire d'inclinaison de la première pièce (2) par rapport à la seconde pièce (3) d'environ 5 à 7° et la seconde phase du mouvement d'inclinaison correspond à une étendue angulaire d'inclinaison de la première pièce (2) par rapport à la seconde pièce (3) d'environ 4 à 6°.

14. Prothèse de disque intervertébral selon l'une quelconque des revendications 1 à 13, **caractérisée en ce que** lesdites surfaces d'articulation (9, 10) de la pièce intermédiaire (4) sont convexes et lesdites surfaces d'articulation respectives (6, 8) des première et seconde pièces (2, 3) sont concaves.

15. Prothèse de disque intervertébral selon l'une quelconque des revendications 1 à 14, **caractérisée en ce que** la surface d'articulation (6) de la première pièce (2) et la surface d'articulation correspondante (9) de la pièce intermédiaire (4) sont des portions de sphère de rayon R 1, et **en ce que** la surface d'articulation (8) de la seconde pièce (3) et la surface d'articulation correspondante (10) de la pièce intermédiaire (4) sont des portions de sphère de rayon R2.

16. Prothèse de disque intervertébral selon la revendication 15, **caractérisée en ce que** le rayon R1 est inférieur au rayon R2.

17. Prothèse de disque intervertébral selon l'une quelconque des revendications 1 à 16, **caractérisée en ce que** la première pièce (2 ; 21) est une pièce supérieure et la seconde pièce (3 ; 22) est une pièce inférieure.

## Claims

1. Intervertebral disk prosthesis comprising:
- first and second parts (2, 3) intended to be attached respectively to two adjacent vertebrae,
- an intermediate part (4) inserted between the first and second parts (2, 3) and comprising articular surfaces (9, 10) that cooperate with respective articular surfaces (6, 8) of the first and second parts (2, 3), and
- means (11, 12) for coupling the first part (2) and the intermediate part (4),
**characterised in that** the coupling means (11, 12) are arranged to leave the intermediate part (4) free relative to the first part (2) during a first phase of a tilting movement of the first part (2) relative to the second part (3), such that the intermediate part (4) is displaced toward an end (E2) of the prosthesis that is opposite to an end (E1) toward which the first part (2) is displaced by tilting relative to the second part (3), and to drive the intermediate part (4) with the first part (2) during a second phase of the tilting movement.

2. Intervertebral disk prosthesis according to claim 1, **characterised in that** said coupling means comprise at least one projecting portion (11) provided on one of the first part (2) and intermediate part (4) and at least one cavity (12) formed in the other of the first part (2) and intermediate part (4), whereby this cavity (12) receives the projecting portion (11) and has a larger section than that of the projecting portion (11).

3. Intervertebral disk prosthesis according to claim 2, **characterised in that** said coupling means comprise a single projecting portion (11) provided on a peripheral part of the first part (2) or the intermediate part (4) and in a sagittal plane.

4. Intervertebral disk prosthesis according to claim 2, **characterised in that** said coupling means comprise first and second projecting portions (28; 48) provided on the first part (21; 41) or the intermediate part (23; 43) and located in a sagittal plane, on both sides of the axis of the prosthesis, and first and second corresponding cavities (29; 49) formed in the intermediate part (23; 43) or the first part (21; 41) respectively.

5. Intervertebral disk prosthesis according to any of claims 1 to 4, **characterised by** further comprising means (13, 15) for coupling the intermediate part (4) and the second part (3), whereby these coupling means limit the relative movements in axial rotation of said intermediate part (4) and second part (3).

6. Intervertebral disk prosthesis according to claim 5, **characterised in that** said means for coupling the intermediate part (4) and the second part (3) comprise a rail (13) extending in a sagittal direction on one of the second part (3) and intermediate part (4) and a groove (15) formed in the other of the second part (3) and intermediate part (4) and cooperating with this rail (13).

7. Intervertebral disk prosthesis according to claim 6, **characterised in that** the rail (13) and the groove (15) define respective inclined support surfaces (14, 16) that cooperate during flexion movements of the prosthesis to slightly raise the center of gravity of the intermediate part (4).

8. Intervertebral disk prosthesis according to claim 5, **characterised in that** said means for coupling the intermediate part (23) and the second part (22) comprise a first groove (30; 30a; 30b) formed in the second part (22) and oriented in a sagittal direction, a second groove (31) formed in the intermediate part (23) and oriented in a frontal direction, and a mobile guide part (32; 32a) cooperating with the first and second grooves (30; 30a; 30b, 31).

9. Intervertebral disk prosthesis according to claim 8, **characterised in that** a clearance (30') exists between the guide part (32) and at least one of the first and second grooves (30, 31), allowing axial rotation play of the intermediate part (23) relative to the second part (22).

10. Intervertebral disk prosthesis according to claim 5, **characterised in that** said means for coupling the intermediate part (43) and the second part (42) comprise first and second studs (50) provided on the second part (42) or the intermediate part (43) and located in a frontal plane, on both sides of the axis of the prosthesis, and third and fourth cavities (51) formed in the intermediate part (43) or the second part (42), respectively, and receiving the studs (50).

11. Intervertebral disk prosthesis according to any of claims 1 to 10, **characterised in that** the first phase of the tilting movement corresponds to about 40 to 60% of the maximum tilting angle of the first part (2) relative to the second part (3), and the second phase corresponds to the remaining 60 to 40%.

12. Intervertebral disk prosthesis according to any of claims 1 to 11, **characterised in that** the first phase of the tilting movement corresponds to an angular tilting range of the first part (2) relative to the second part (3) of about 4 to 8°, and the second phase of the tilting movement corresponds to an angular tilting range of the first part (2) relative to the second part (3) of about 3 to 7°.

13. Intervertebral disk prosthesis according to any of claims 1 to 11, **characterised in that** the first phase of the tilting movement corresponds to an angular tilting range of the first part (2) relative to the second part (3) of about 5 to 7°, and the second phase of the tilting movement corresponds to an angular tilting range of the first part (2) relative to the second part (3) of about 4 to 6°.

14. Intervertebral disk prosthesis according to any of claims 1 to 13, **characterised in that** said articular surfaces (9, 10) of the intermediate part (4) are convex, and said respective articular surfaces (6, 8) of the first and second parts (2, 3) are concave.

15. Intervertebral disk prosthesis according to any of claims 1 to 14, **characterised in that** the articular surface (6) of the first part (2) and the corresponding articular surface (9) of the intermediate part (4) are spherical portions of radius R1, and **in that** the articular surface (8) of the second part (3) and the corresponding articular surface (10) of the intermediate part (4) are spherical portions of radius R2.

16. Intervertebral disk prosthesis according to claim 15, **characterised in that** the radius R1 is less than the radius R2.

17. Intervertebral disk prosthesis according to any of claims 1 to 16, **characterised in that** the first part (2; 21) is an upper part, and the second part (3; 22) is a lower part.

## Patentansprüche

1. Bandscheibenprothese, welche umfasst:
- ein erstes und ein zweites Teil (2, 3), die dazu bestimmt sind, jeweils an einem von zwei benachbarten Wirbeln befestigt zu werden,
- ein Zwischenteil (4), das zwischen dem ersten und dem zweiten Teil (2, 3) angeordnet ist und zwei Gelenkflächen (9, 10) umfasst, die mit jeweiligen Gelenkflächen (6, 8) des ersten und zweiten Teils (2, 3) zusammenwirken, und
- Kopplungsmittel (11, 12) zur Kopplung des ersten Teils (2) und des Zwischenteils (4),
**dadurch gekennzeichnet, dass** die Kopplungsmittel (11, 12) dazu eingerichtet sind, während einer ersten Phase einer Neigungsbewegung des ersten Teils (2) bezüglich des zweiten Teils (3) das Zwischenteil (4) frei bezüglich des ersten Teils (2) zu lassen, derart, dass das Zwischenteil (4) sich zu einem Ende (E2) der Prothese hin bewegt, das einem Ende (E1) gegenüberliegt, zu dem sich das erste Teil (2) hinbewegt, indem es sich bezüglich des zweiten Teils (3) neigt, und während einer zweiten Phase der Neigungsbewegung das Zwischenteil (4) mit dem ersten Teil (2) mitzunehmen.

2. Bandscheibenprothese nach Anspruch 1, **dadurch gekennzeichnet, dass** die Kopplungsmittel mindestens einen Vorsprung (11), der an einem von dem ersten Teil (2) und dem Zwischenteil (4) vorgesehen ist, und mindestens eine Vertiefung (12), die in dem anderen von dem ersten Teil (2) und dem Zwischenteil (4) ausgebildet ist, umfassen, wobei diese Vertiefung (12) den Vorsprung (11) aufnimmt und einen größeren Querschnitt als der Vorsprung (11) aufweist.

3. Bandscheibenprothese nach Anspruch 2, **dadurch gekennzeichnet, dass** die Kopplungsmittel einen einzigen Vorsprung (11) umfassen, der an einem Umfangsabschnitt des ersten Teils (2) oder des Zwischenteils (4) und in einer Sagittalebene vorgesehen ist.

4. Bandscheibenprothese nach Anspruch 2, **dadurch gekennzeichnet, dass** die Kopplungsmittel einen ersten und einen zweiten Vorsprung (28; 48), die an dem ersten Teil (21; 41) oder dem Zwischenteil (23; 43) vorgesehen sind und sich in einer Sagittalebene beiderseits der Achse der Prothese befinden, und eine erste und eine zweite entsprechende Vertiefung (29; 49), die in dem Zwischenteil (23; 43) bzw. dem ersten Teil (21; 41) ausgebildet sind, umfassen.

5. Bandscheibenprothese nach einem der Ansprüche 1 bis 4, **dadurch gekennzeichnet, dass** sie außerdem Kopplungsmittel (13, 15) zur Kopplung des Zwischenteils (4) und des zweiten Teils (3) umfasst, welche die relativen axialen Drehbewegungen des Zwischenteils (4) und des zweiten Teils (3) begrenzen.

6. Bandscheibenprothese nach Anspruch 5, **dadurch gekennzeichnet, dass** die Kopplungsmittel zur Kopplung des Zwischenteils (4) und des zweiten Teils (3) eine Schiene (13), die sich in einer sagittalen Richtung an einem von dem zweiten Teil (3) und dem Zwischenteil (4) erstreckt, und eine Nut (15), die in dem anderen von dem zweiten Teil (3) und dem Zwischenteil (4) ausgebildet ist und mit dieser Schiene (13) zusammenwirkt, umfassen.

7. Bandscheibenprothese nach Anspruch 6, **dadurch gekennzeichnet, dass** die Schiene (13) und die Nut (15) jeweilige geneigte Stützflächen (14, 16) definieren, welche bei Biegebewegungen der Prothese zusammenwirken, um den Schwerpunkt des Zwischenteils (4) leicht nach oben zu verlagern.

8. Bandscheibenprothese nach Anspruch 5, **dadurch gekennzeichnet, dass** die Kopplungsmittel des Zwischenteils (23) und des zweiten Teils (22) eine erste Nut (30; 30a; 30b), die in dem zweiten Teil (22) ausgebildet ist und in einer sagittalen Richtung verläuft, eine zweite Nut (31), die in dem Zwischenteil (23) ausgebildet ist und in einer frontalen Richtung verläuft, und ein bewegliches Führungsteil (32; 32a), das mit der ersten und der zweiten Nut (30; 30a; 30b; 31) zusammenwirkt, umfassen.

9. Bandscheibenprothese nach Anspruch 8, **dadurch gekennzeichnet, dass** ein Spiel (30') zwischen dem Führungsteil (32) und wenigstens einer von der ersten und der zweiten Nut (30, 31) vorhanden ist, das ein axiales Verdrehspiel des Zwischenteils (23) bezüglich des zweiten Teils (22) ermöglicht.

10. Bandscheibenprothese nach Anspruch 5, **dadurch gekennzeichnet, dass** die Kopplungsmittel des Zwischenteils (43) und des zweiten Teils (42) einen ersten und einen zweiten Zapfen (50), die an dem zweiten Teil (42) oder dem Zwischenteil (43) vorgesehen sind und sich in einer frontalen Ebene beiderseits der Achse der Prothese befinden, und eine dritte und eine vierte Vertiefung (51), die in dem Zwischenteil (43) bzw. dem zweiten Teil (42) ausgebildet sind und die Zapfen (50) aufnehmen, umfassen.

11. Bandscheibenprothese nach einem der Ansprüche 1 bis 10, **dadurch gekennzeichnet, dass** die erste Phase der Neigungsbewegung ungefähr 40 bis 60 % des maximalen Neigungswinkels des ersten Teils (2) bezüglich des zweiten Teils (3) entspricht und die zweite Phase den übrigen ungefähr 60 bis 40 % entspricht.

12. Bandscheibenprothese nach einem der Ansprüche 1 bis 11, **dadurch gekennzeichnet, dass** die erste Phase der Neigungsbewegung einer Winkelerstreckung der Neigung des ersten Teils (2) bezüglich des zweiten Teils (3) von ungefähr 4 bis 8° entspricht und die zweite Phase der Neigungsbewegung einer Winkelerstreckung der Neigung des ersten Teils (2) bezüglich des zweiten Teils (3) von ungefähr 3 bis 7° entspricht.

13. Bandscheibenprothese nach einem der Ansprüche 1 bis 11, **dadurch gekennzeichnet, dass** die erste Phase der Neigungsbewegung einer Winkelerstreckung der Neigung des ersten Teils (2) bezüglich des zweiten Teils (3) von ungefähr 5 bis 7° entspricht und die zweite Phase der Neigungsbewegung einer Winkelerstreckung der Neigung des ersten Teils (2) bezüglich des zweiten Teils (3) von ungefähr 4 bis 6° entspricht.

14. Bandscheibenprothese nach einem der Ansprüche 1 bis 13, **dadurch gekennzeichnet, dass** die Gelenkflächen (9, 10) des Zwischenteils (4) konvex sind und die jeweiligen Gelenkflächen (6, 8) des ersten und des zweiten Teils (2, 3) konkav sind.

15. Bandscheibenprothese nach einem der Ansprüche 1 bis 14, **dadurch gekennzeichnet, dass** die Gelenkfläche (6) des ersten Teils (2) und die entsprechende Gelenkfläche (9) des Zwischenteils (4) Kugelabschnitte mit einem Radius R1 sind, und **dadurch**, dass die Gelenkfläche (8) des zweiten Teils (3) und die entsprechende Gelenkfläche (10) des Zwischenteils (4) Kugelabschnitte mit einem Radius R2 sind.

16. Bandscheibenprothese nach Anspruch 15, **dadurch gekennzeichnet, dass** der Radius R1 kleiner als der Radius R2 ist.

17. Bandscheibenprothese nach einem der Ansprüche 1 bis 16, **dadurch gekennzeichnet, dass** das erste Teil (2; 21) ein oberes Teil ist und das zweite Teil (3; 23) ein unteres Teil ist.
